Europäisches Patentamt

European Patent Office

Office européen des brevets

(11) Veröffentlichungsnummer: **0 455 036 A2**

# EUROPÄISCHE PATENTANMELDUNG

(21) Anmeldenummer: 91105983.0

(22) Anmeldetag: 15.04.91

(51) Int. Cl.5: **A61F 2/06**

(30) Priorität: 03.05.90 DE 9005035 U

(43) Veröffentlichungstag der Anmeldung:
**06.11.91 Patentblatt 91/45**

(84) Benannte Vertragsstaaten:
**DE FR GB**

(71) Anmelder: **W.L. Gore & Associates GmbH**
**Hermann-Oberth-Strasse 22**
**W-8011 Putzbrunn(DE)**

(72) Erfinder: **Kramer, Valentin**
**Flurstrasse 3**
**8152 Feldkirchen-Westerham(DE)**
Erfinder: **Franklin, Joao**
**Rua Gregorio Lopes Lote 1597-11E, Restelo**
**1400 Lisboa(PT)**

(74) Vertreter: **Klunker . Schmitt-Nilson . Hirsch**
**Winzererstrasse 106**
**W-8000 München 40(DE)**

(54) **Vorrichtung zur Verwendung in der Gefässchirurgie.**

(57) Die Erfindung betrifft eine Vorrichtung zur Verwendung in der Gefäßchirurgie mit einem Hohlzylinder (10), der nur in axialer Richtung durchlässig ist, dessen eines Ende mit einem ringförmigen Ansatz (30), der größer ist als der Außendurchmesser des Hohlzylinders (10), versehen ist, und auf dessen Mantelfläche kegelstumpfförmige Rückhaltemittel (40) vorgesehen sind, um die Vorrichtung (5) zu fixieren. Zur Fixierung der Vorrichtung kann auch eine elastische Scheibe (60) auf die Vorrichtung (5) aufgeschoben werden. Die elastische Scheibe (60) verspannt die Vorrichtung (5) dann gegenüber der Gefäßwand (50), so daß die Vorrichtung (5) sicher in der Gefäßwand (50) fixiert ist.

Fig. 1

Die Erfindung betrifft eine Vorrichtung zur Verwendung in der Gefäß-Wiederherstellungschirurgie, die beispielsweise Blutgefäße mit Prothesen oder Prothesen miteinander verbindet.

Die operative Behandlung von Gefäßkrankheiten hat stark zugenommen. In vielen Fällen ist es erforderlich, Kunststoffprothesen einzusetzen, um das erkrankte Gefäß zu ersetzen oder zu umgehen. Dieses Vorgehen kann eine End-zu-End-, oder beide Arten der Verbindung, üblicherweise als Gefäßanastomose bezeichnet, erfordern.

Die herkömmliche Methode der Gefäßanastomose ist ein Nähverfahren mit sehr dünnem Faden. Eine solche Präzisionsarbeit ist zeitaufwendig. Andererseits sollte bei einer solchen Operation der Zeitaufwand auf ein Minimum beschränkt werden, um Komplikationen aufgrund der Unterbrechung der Blutzirkulation während der Anastomose zu minimieren. Dies ist besonders bei komplizierten Operationen, welche lebenswichtige Organe, z.B. Nieren, Leber usw. betreffen, sehr wichtig.

Der Erfindung liegt die Aufgabe zugrunde, eine Vorrichtung zu schaffen, mit der der Zeitaufwand zur Gefäßanastomose reduziert wird, und die nach dem Einsetzen nicht vom Körper abgestoßen wird.

Diese Aufgabe wird erfindungsgemäß durch eine Vorrichtung, wie sie in Anspruch 1 gekennzeichnet ist, gelöst. Damit wird eine deutliche Reduzierung der Operationsdauer erreicht, da ein Nähen vermieden wird. Zusätzlich kann der Blutverlust beträchtlich verringert werden, da keine Stechkanalblutung auftritt. Bei der Operation kann auf aufwendige Werkzeuge verzichtet werden. Die Vorrichtung wird einfach in eine bestimmte Öffnung des Blutgefäßes der Prothese eingesetzt und ist dann dort fixiert. Solche Vorrichtungen sind insbesondere auch proximal einsetzbar, da sie hohe Drücke ertragen können.

In einer Weiterbildung der Erfindung für End-zu-Seit-Anastomose umfaßt die Vorrichtung zusätzlich eine elastische Scheibe, die auf die mit mindestens einem Rückhaltemittel versehene Mantelfläche ausgehend von der anderen Stirnseite aufschiebbar ist, nachdem die Vorrichtung in eine geeignete Öffnung in einer Prothese eingesetzt worden ist, wobei die Scheibe in axialer Richtung einerseits durch das/die Rückhaltemittel und andererseits durch die Prothese, die sich an dem trompetenförmigen Ansatz abstützt, festgelegt ist und die Vorrichtung dadurch in einer geeigneten Öffnung fixierbar ist. Durch diese Maßnahme wird eine besonders zuverlässige Verbindung auf einfachste Weise möglich. Die Vorrichtung wird, wie bereits beschrieben, in eine Öffnung in der Prothese eingesetzt und eine elastische Scheibe wird soweit von außen darübergeschoben, bis die Scheibe fest auf dem Blutgefäß aufsitzt und durch ein Rückhaltemittel in der Prothese gehalten wird. Auf diese Weise wird die Vorrichtung gegenüber der Prothese verspannt und zuverlässig fixiert.

Andere Weiterbildungen der Erfindung sind in den Ansprüchen 3 bis 8 beschrieben.

Darüberhinaus wird die Aufgabe erfindungsgemäß durch eine Vorrichtung, wie sie in Anspruch 9 gekennzeichnet ist, gelöst. Diese Vorrichtung ist besonders geeignet zur einfachen anastomatischen Verbindung zweier Hohlorgane. Das jeweilige Hohlorgan wird auf diese Vorrichtung aufgeschoben und durch eine darum gelegte Schlinge, welche festgezogen wird, fixiert. Die Schlinge liegt dabei im Bereich der Nut. Mit dieser Vorrichtung kann der Zeitaufwand bei einer Operation und die Verwirbelung an der Schnittstelle deutlich verringert werden.

Andere Weiterbildungen der Erfindung sind in den Unteransprüchen 10 bis 13 beschrieben.

Weitere Einzelheiten, Merkmale, Vorteile und Anwendungsmöglichkeiten ergeben sich aus den in den Zeichnungen dargestellten Ausführungsbeispielen, die nachfolgend näher beschrieben werden. Es zeigen:

Fig. 1    die erfindungsgemäße Vorrichtung zur End-zu-Seit-Anastomose in Seitenansicht und in Draufsicht;

Fig. 2    die in einem Blutgefäß eingesetzte Vorrichtung aus Fig. 1;

Fig. 3    die in Fig. 2 gezeigte Vorrichtung, die mit einer elastischen Scheibe zusätzlich fixiert ist;

Fig. 4    die in Fig. 3 gezeigte elastische Scheibe in entspanntem Zustand;

Fig. 5    eine Vorrichtung zur End-zu-End-Anastomose in Seitenansicht und in Draufsicht;

Fig. 6    die in Fig. 1 und Fig. 5 gezeigten Vorrichtungen, wenn sie in einem Blutgefäß /einer Prothese eingesetzt sind; und

Fig. 7    die Vorbereitung einer Prothese zur End-zu-Seit-Anastomose.

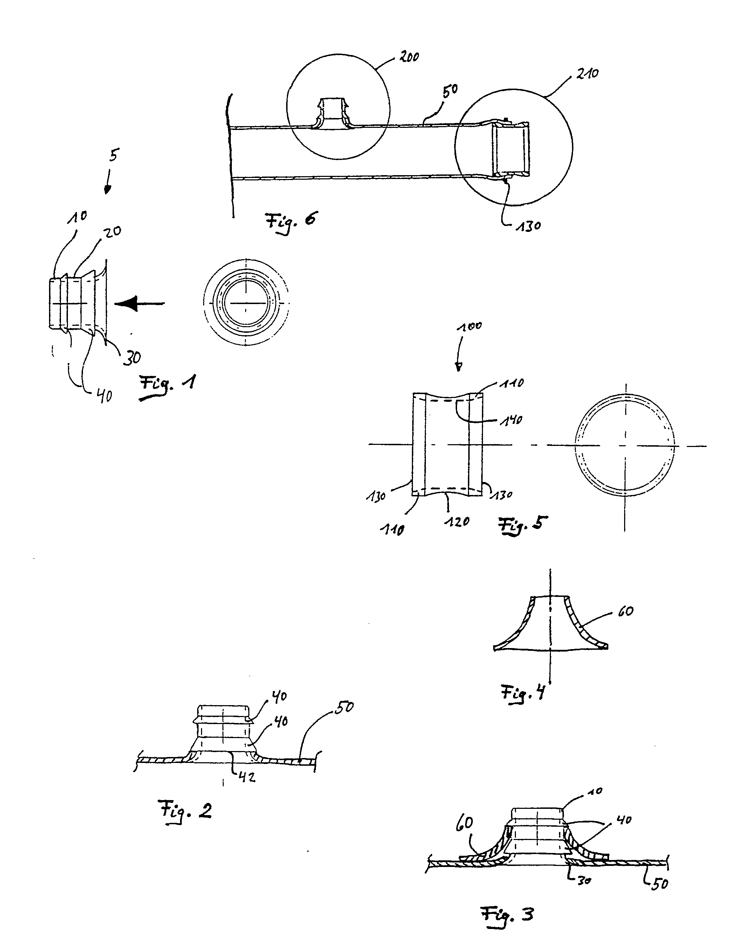

Die in Fig. 1 dargestellte erfindungsgemäße Vorrichtung 5 zur Verwendung in der Gefäßchirurgie weist einen Hohlzylinder 10 auf, der an einem Ende 30 trompetenförmig erweitert ist. Das Material der Vorrichtung ist Polytetrafluorethylen (PTFE), da die Wahrscheinlichkeit,
daß
es vom Körper abgestoßen wird, sehr gering ist. Im eingebauten Zustand (siehe Fig. 6) strömt Blut bei dem trompetenförmigen Ende 30 in die Vorrichtung ein und wird dort entlang der Achse durch die Vorrichtung strömen. Der Blutstrom kann auf diese Weise verzweigt werden.

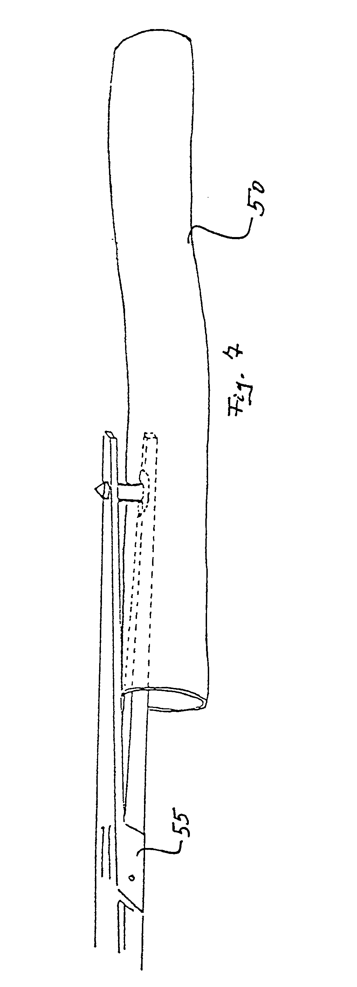

Auf der Mantelfläche 20 des Hohlzylinders 10 sind zwei Rückhaltemittel 40 angeordnet, die kegelstumpfförmig ausgebildet sind. Sie sind so angeordnet, daß deren jeweilige Basis 42 einen kleineren axialen Abstand von dem trompetenförmigen Ansatz 30 aufweist als die jeweilige imaginäre Spitze des entsprechenden Kegels. Wird die Vorrichtung in der in Fig. 1 gezeigten Pfeilrichtung in die Gefäßwand 50 eingeschoben, so wird sie dort durch eines der Rückhaltemittel 40, fixiert (Fig. 2). Die Gefäßwand 50 steht dabei mit der Basis 42 des Rückhaltemittels 40 in

Eingriff und verhindert dadurch eine entgegen der in Fig. 1 gezeigten Pfeilrichtung gerichtete Bewegung. Vorteilhafterweise weisen die Rückhaltemittel 40 unterschiedliche geometrische Gestaltungen auf. So ist das in Fig. 2 gezeigte obere Rückhaltemittel 40 ein hohler Kegelstumpf mit kleinerer Höhe und kleinerer Basis als der darunter befindliche mit der Gefäßwand 50 zusammenwirkende.

In Fig. 7 wird die Vorbereitung einer Prothese mit einem Werkzeug 55 gezeigt. Ein Dorn mit definierter Geometrie wird dabei durch die Gefäßwand 50 getrieben und schafft dadurch die für die Vorrichtung erforderliche Öffnung.

Ist die Vorrichtung 5 in der beschriebenen Weise in das Blutgefäß 50 eingesetzt worden, so kann sie zusätzlich noch durch eine elastische Scheibe 60 fixiert werden. Diese Scheibe 60 weist einen Innendurchmesser auf, der geringfügig größer ist als der Durchmesser der Mantelfläche ist, aber kleiner als der Außendurchmesser der ringförmigen Rückhaltemittel 40. Wird diese Scheibe 60 nun über die Vorrichtung 5 geschoben, so rastet sie jeweils an der dem trompetenförmigen Ansatz 30 gegenüberliegenden Basis 42 des Rückhaltemittels 40 ein, wenn sie mit etwas Kraft darübergeschoben wird. Auf diese Weise wird eine Vorspannung zwischen der Gefäßwand 50, der Vorrichtung 5 und der Scheibe 60 geschaffen, die eine besonders dauerhafte Fixierung der Vorrichtung in dem Blutgefäß gewährleistet. In Fig. 4 ist die elastische Scheibe in unverformtem Zustand gezeigt, während die Scheibe in Fig. 3 in verformtem, eingebautem Zustand gezeigt wird.

Für eine sichere Fixierung der Vorrichtung 5 in der Prothese ist es nicht erforderlich, daß die Gefäßwand 50 sich an dem Rückhaltemittel 40 abstützt. Die Fixierung ausschließlich mit der Scheibe 60 reicht für eine sichere Fixierung vollkommen aus.

Die in Fig. 5 gezeigte Vorrichtung dient zur End-zu-End-Anastomose. Die zu verbindenden Hohlorgane werden dabei auf die Vorrichtung von beiden Seiten aufgeschoben, so daß sie den zylindrischen Abschnitt 110 und den nutförmigen Abschnitt 120 zumindest teilweise umschließen. In Fig. 6 wird eine solche Verbindung an einem Blutgefäß durch den Kreis 210 gekennzeichnet. Die Fixierung des Blutgefäßes auf der Vorrichtung 100 wird durch eine pTFE-Schlinge 130, die festgezogen wird, erreicht. Der Innendurchmesser der Vorrichtung 100 weist einen Mittelteil 140, der hohlzylinderförmig ist, auf, wobei die Randbereiche sich konisch erweitern, so daß die Strömung innerhalb der Vorrichtung nur minimal beeinträchtigt wird. Auf diese Weise können verschiedene Blutgefäße und/oder Prothesen miteinander verbunden werden.

Fig. 6 zeigt die erfindungsgemäßen Vorrichtungen zur End-zu-End-Anastomose 210 und zur End-zu-Seit-Anastomose 200.

**Patentansprüche**

1. Vorrichtung (5) zur Verwendung in der Gefäßchirurgie,
   **gekennzeichnet** durch einen Hohlkörper, der im wesentlichen die Form eines Hohlzylinders (10) aufweist, wobei der Hohlzylinder (10)
   - nur in axialer Richtung durchlässig ist,
   - an einem Ende mit einem trompetenförmigen Ansatz (30), dessen Außendurchmesser größer ist als der Außendurchmesser des Hohlzylinders (10), versehen ist,
   - und auf dessen Mantelfläche (20) mindestens ein Rückhaltemittel (40) vorgesehen ist, so daß die Vorrichtung (5) in eine geeignete Öffnung mit geringem Kraftaufwand einschiebbar ist, dort fixiert bleibt und nur mit einem erheblich größeren Kraftaufwand wieder aus der Öffnung entfernbar ist.

2. Vorrichtung (5) nach Anspruch 1,
   dadurch **gekennzeichnet,** daß
   - die Vorrichtung (5) zusätzlich eine elastische Scheibe (60) umfaßt,
   - die auf die mit mindestens einem Rückhaltemittel (40) versehene Mantelfläche (20) aufschiebbar ist, nachdem die Vorrichtung (5) in eine geeignete Öffnung in einer Gefäßwand (50) eingesetzt worden ist,
   - die Scheibe (60) in axialer Richtung einerseits durch das/die Rücknaltemittel (40) und andererseits durch die Gefäßwand (50), die sich an dem trompetenförmigen Ansatz (30) abstützt, festgelegt ist.

3. Vorrichtung (5) nach Anspruch 1 oder 2,
   dadurch **gekennzeichnet,** daß das/die Rückhaltemittel (40) ringförmig auf der Mantelfläche (20) des Hohlzylinders (10) angeordnet sind.

4. Vorrichtung (5) nach Anspruch 3,
   dadurch **gekennzeichnet,** daß das/die Rückhaltemittel (40) kegelstumpfförmig sind, wobei die Basis (42) des jeweiligen Kegelstumpfes in kleinerem axialen Abstand zu dem trompetenförmigen Ansatz (30) angeordnet ist als die imaginäre Kegelstumpfspitze.

5. Vorrichtung (5) nach einem der Ansprüche 3 bis 4,
dadurch **gekennzeichnet**, daß das/die ringförmig angeordneten Rückhaltemittel (40) in axialer Richtung versetzt auf der Mantelfläche (20) des Hohlzylinders (10) angeordnet sind.

6. Vorrichtung (5) nach Anspruch 5,
dadurch **gekennzeichnet**, daß die axial versetzten ringförmig angeordneten Rückhaltemittel (40) unterschiedliche Außendurchmesser aufweisen.

7. Vorrichtung (5) nach einem der vorhergehenden Ansprüche,
dadurch **gekennzeichnet**, daß das/die Rückhaltemittel (40) widerhakenähnliche Form haben.

8. Vorrichtung (5) nach einem der vorhergehenden Ansprüche,
dadurch **gekennzeichnet**, daß der trompetenförmige Ansatz (30) im Querschnitt eine wellenförmige Gestaltung aufweist.

9. Vorrichtung (100) zur Verwendung in der Gefäßchirurgie,
**gekennzeichnet** durch einen Hohlkörper, der im wesentlichen die Form eines Hohlzylinders aufweist, wobei der Hohlzylinder nur in axialer Richtung durchlässig ist, die Mantelfläche des Hohlzylinders eine Nut (120) aufweist, und der Innendurchmesser des Hohlzylinders sich jeweils zu den beiden Stirnseiten des Hohlzylinders hin konisch erweitert.

10. Vorrichtung (100) nach Anspruch 9,
dadurch **gekennzeichnet**, daß die Nut (120) mittig zwischen den beiden Stirnseiten (130) des Hohlzylinders angeordnet ist und/oder der Hohlzylinder zusätzlich einen zylinderförmigen Innenabschnitt (140) aufweist.

11. Vorrichtung nach einem der vorhergehenden Ansprüche,
dadurch **gekennzeichnet**, daß die Innenflächen des Hohlzylinders glatt sind, ohne scharfe Winkel.

12. Vorrichtung nach einem der vorhergehenden Ansprüche,
dadurch **gekennzeichnet**, daß sie aus biokompatiblem inertem Material besteht.

13. Vorrichtung nach einem der vorhergehenden Ansprüche,
dadurch **gekennzeichnet**, daß sie ausschließlich aus PTFE-Material besteht.

14. Vorrichtung nach einem der vorhergehenden Ansprüche,
dadurch **gekennzeichnet**, daß der Innendurchmesser des Hohlzylinders mindestens 4 mm beträgt, vorzugsweise mindestens 6 mm.

Fig. 6

Fig. 1

Fig. 5

Fig. 4

Fig. 2

Fig. 3

Fig. 4